# EUROPEAN PATENT SPECIFICATION

(11) **EP 2 976 058 B1**
(45) Date of publication and mention of the grant of the patent: **08.08.2018**
(21) Application number: 13732260.8
(22) Date of filing: 19.03.2013
(51) Int. Cl.: A61K 8/27, A61Q 11/00, A61K 8/02, A61K 8/04

(54) **TWO-PHASE DENTIFRICES**
ZWEIPHASIGE ZAHNPASTEN
DENTIFRICES À DEUX PHASES

(43) Date of publication of application: 27.01.2016
(73) Proprietor: Colgate-Palmolive Company, New York, NY 10022 (US)
(72) Inventor: POTNIS, Shashank, Thane 400610 Maharashtra (IN); SUBRAMANYAM, Ravi, Mumbai 400076 Maharashtra (IN); LEWIS, Navin, Mumbai 400031 Maharashtra (IN)
(74) Representative: Wibbelmann, Jobst
(86) International application number: PCT/IN2013/000184
(87) International publication number: WO 2014/147630

(56) References cited:
- EP-A1- 0 740 932
- WO-A1-99/01342
- WO-A1-2006/050777
- US-A- 4 487 757
- US-A- 5 616 313
- US-A- 6 121 315

## Description

### BACKGROUND

Soluble zinc salts, such as zinc citrate, have been used in dentifrice compositions, see, e.g., U.S. Patent No. 6,121,315, but may react with common excipients in dentifrice to form insoluble salts and so reduce delivery of the zinc to the tooth surface. For example, many people prefer dentifrice comprising calcium carbonate (chalk) abrasive, but zinc ions will react with calcium carbonate to form insoluble zinc carbonate, thereby reducing the delivery of the zinc to the tooth surface.

Thus, there is a need for compositions which are able to overcome the problems with formulation zinc salts in a calcium carbonate backbone.

WO-A-2006/050777 discloses a toothpaste composition. US-A-5,616,313 discloses a method for treating gingival and periodontal tissues. WO-A-99/01342 discloses an apparatus for inserting plural materials into containers

### SUMMARY

The present invention provides a toothpaste product according to claim 1, a striped toothpaste according to claim 11, and a method of making a dentifrice according to claim 12. Preferred features are defined in the dependent claims. The present invention also provides a composition of the invention for use in providing an antibacterial benefit to the oral cavity of a subject in need thereof.

Some embodiments of the present invention provide enhanced delivery of Zn⁺⁺ ions from a zinc ion source such as ZnO and/or Zn citrate to the tooth surface using a calcium carbonate dentifrice, thus providing enhanced antibacterial efficacy and enhanced protection against erosion and hypersensitivity. The zinc ion source is incorporated in silica dentifrice stripes entrained in a calcium carbonate based dentifrice formulation, providing the benefits and consumer acceptability of a calcium carbonate dentifrice. This formulation provides enhanced delivery of zinc to the tooth surface compared to simply incorporating the zinc into the calcium carbonate dentifrice, and thus enhanced the antibacterial, antisensitivity and antierosion benefits of the zinc. Products comprising the toothpaste and methods of making and using the dentifrice are also provided.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiments) is merely exemplary in nature and is in no way intended to limit the invention, its application, or uses.

The invention provides, in one embodiment, formulations to enhance the delivery of Zn⁺⁺ ions from a zinc ion source, e.g., zinc oxide and/or zinc citrate, onto hard tissue surfaces e.g., hydroxyapatite (HAP), from a calcium carbonate dentifrice, to obtain enhanced antibacterial efficacy. Zinc ions have good antibacterial efficacy compared to conventional chalk-based formulations, but when zinc ions are provided in calcium carbonate formulations, we have found that the delivery of zinc to the tooth surface is relatively poor. In this formulation, a zinc ion source is incorporated in silica gel stripes entrained in a calcium carbonate based dentifrice. This formulation surprisingly enhances the delivery of zinc ion on hard tissues in the oral cavity, thus providing enhanced antibacterial benefits compared to formulations containing zinc ion sources in a calcium carbonate matrix.

The invention thus provides a toothpaste product (Product 1) comprising
a first dentifrice comprising a calcium carbonate abrasive,
a second dentifrice comprising a zinc ion source in a gel base, and
a container which holds the first dentifrice physically separate from the second dentifrice until the dentifrices are dispensed, and which is capable of dispensing the second dentifrice as a stripe entrained in the first dentifrice,
wherein the amount of first dentifrice relative to the second dentifrice is 4:1.

For example, Product 1 includes, e.g.,
1.1. Product 1 wherein the zinc ion source is selected from zinc oxide, zinc citrate, and mixtures thereof.
1.2. Product 1 or 1.1 wherein the amount of first dentifrice relative to the second dentifrice is 4:1.
1.3. Any of the foregoing products wherein the first dentifrice is substantially free of zinc.
1.4. Any of the foregoing products wherein the second dentifrice is substantially free of calcium.
1.5. Any of the foregoing products wherein the total amount of zinc in the first and second dentifrice combined is from 0.1-2%, e.g., 0.5-1.5%, e.g., about 0.8%.
1.6. Any of the foregoing products wherein the second dentifrice contains zinc in an amount of 0.1-10%, e.g., 2-6%, e.g. about 4%.
1.7. Any of the foregoing products wherein the second dentifrice contains a zinc ion source selected from (i) zinc oxide in an amount of 4-6%, e.g., about 5%, and (ii) zinc oxide in an amount of 2-3%, e.g., about 2.5% and zinc citrate in an amount of 8-12%, e.g., about 10%.
1.8. Any of the foregoing products wherein the ratio of the first dentifrice to the second dentifrice is 4:1 and the second dentifrice contains about 5% zinc oxide.
1.9. Any of the foregoing products wherein the second dentifrice contains a viscosity modifying amount of one or more thickening agents selected from thickening silica, polyethylene glycols, polysaccharides (e.g., cellulose derivatives, for example carboxymethyl cellulose, polysaccharide gums, for example xanthan gum or carrageenan gum), and combinations thereof; e.g., selected from carboxymethyl cellulose, xanthan, thickening silica, and mixtures thereof.
1.10. Any of the foregoing products wherein the calcium carbonate abrasive in the first dentifrice is a mixture of precipitated calcium carbonate and natural calcium carbonate, e.g., in an amount of 35-45% by weight of the first dentifrice.
1.11. Any of the foregoing products wherein the second dentifrice comprises silica abrasive, e.g. 10-40%, e.g. about 20%.
1.12. Any of the foregoing products wherein the first dentifrice is an opaque white paste and the second abrasive is a colored translucent gel.
1.13. Any of the foregoing products wherein the first and/or second dentifrice comprise an effective amount of fluoride, e.g., sodium monofluorophosphate in an amount of 0.5-1% by weight of the first and second dentifrice combined..
1.14. Any of the foregoing products wherein the first dentifrice further comprises one or more of one or more of water, abrasives in addition to calcium carbonate, surfactants, foaming agents, vitamins, polymers, enzymes, humectants, thickeners, antimicrobial agents, preservatives, flavorings, colorings and/or combinations thereof.
1.15. Any of the foregoing products wherein the second dentifrice further comprises one or more of one or more of water, abrasives other than calcium carbonate, surfactants, foaming agents, vitamins, polymers, enzymes, humectants, thickeners, antimicrobial agents, preservatives, flavorings, colorings and/or combinations thereof.
1.16. Any of the foregoing products wherein the first dentifrice comprises one or more or all of the following ingredients:

| Ingredients (amounts given as w/w% of first dentifrice of Product 1) | Range | For example, about |
|---|---|---|
| Sorbitol 70 % | 20-25 | 21 |
| Xanthan | 0-0.5 | 0.2 |
| Carboxymethylcellulose | 0.5-1.0 | 0.7 |
| Sodium Chloride | 0-0.8 | 0.6 |
| Sodium Saccharin | 0.25-0.45 | 0.3 |
| Sodium Silicate | 0-1.5 | 1.2 |
| Sodium Bicarbonate | 0-1.0 | 0.6 |
| Thickenin Silica | 1-2.5 | 2.5 |
| Calcium Carbonate | 35-45 | 40 |
| Tetrasodium pyrophosphate | 0-1 | 0.5 |
| Sodium Monofluorophosphate | 0.5-1 | 0.76 |
| Sodium Lauryl Sulfate Granules | 1.2-2.5 | 2.2 |
| Flavor | 1-1.5 | 1 |
| Purified Water | 20-40 | 25-30, or q.s |

1.17. Any of the foregoing products wherein the second dentifrice comprises one or more or all of the following ingredients:

| Amounts given as w/w% of second dentifrice of Product 1 | Range | For example, about |
|---|---|---|
| Sorbitol 70 % | 30-50 | 40 |
| Xanthan | 0-0.5 | 0.2 |
| Carboxymethylcellulose | 0.5-1.0 | 0.72 |
| Sodium Saccharin | 0.25-0.45 | 0.27 |
| Thickening Silica | 1-2.5 | 1.5 |
| Abrasive Silica | 10-40 | 20 |
| Sodium Monofluorophosphate | 0.5-1 | 0.76 |
| Sodium Lauryl Sulfate Granules | 1.2-2.5 | 2.25 |
| Flavor | 1-1.5 | 1 |
| Purified Water | 20-40 | 25-30, or q.s. |
| Zinc ion | 0.5-10 | 4% (e.g., corresponding to 5% ZnO) |
| Pigment | 0-0.1 | 0.05 |

Also disclosed are methods to (i) reduce hypersensitivity of the teeth, (ii) to reduce plaque accumulation, (iii) reduce or inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; and/or (xv) promote systemic health, including cardiovascular health, e.g., by reducing potential for systemic infection via the oral tissues, in a patient in need thereof, comprising brushing the teeth with the dentifrice from Product 1, et seq.

The invention further provides a striped toothpaste comprising a first dentifrice (e.g., as described above for Product 1, et seq.) comprising a calcium carbonate abrasive and a second dentifrice (e.g., as described above for Product 1, et seq.), comprising a zinc ion source in a gel base, wherein the amount of first dentifrice relative to the second dentifrice is from 3:1 to 5:1, wherein the second dentifrice is entrained as a stripe in the first dentifrice.

The invention further provides a method of making a dentifrice comprising a first dentifrice (e.g., as described above for Product 1, et seq.) comprising a calcium carbonate abrasive and a second dentifrice (e.g., as described above for Product 1, et seq.), comprising a zinc ion source in a gel base, wherein the amount of first dentifrice relative to the second dentifrice is from 3:1 to 5:1, comprising entraining the second dentifrice as a stripe in the first dentifrice.

*Containers:* Containers for dispensing a striped toothpaste such as described herein, e.g., for use Product 1, et seq., will generally have at least two compartments to keep the two dentifrices separate until dispensing. Multi-chamber containers may utilize a dual or multi-chamber tube. These can be, for example, side by side dual chamber tubes as described in U.S. Pat. No. 1,894,115; U.S. Pat. No. 3,758,520 and U.S. Pat. No. 3,980,222 or concentric tubes as described in U.S. Pat. No. 1,535,529; U.S. Pat. No. 1,639,699 and U.S. Pat. No. 1,699,532, and may contain a flow modifying unit in the nozzle of the tube. Exemplary containers for dispensing striped toothpastes as described are described, e.g., in US 4,969,767; US 5941420; US 6,454,130; and US 7,617,950.

*Zinc and Other Active Agents:* "Zinc salts" as used herein refers to zinc ion sources, particularly soluble salts such as zinc citrate. "Zinc salts" for purposes of this application may include zinc oxide, which although not a salt in the strict chemical sense, is commonly referred to in the art as a zinc salt, for ease of reference. The amount of zinc in the formulation will typically be on the order of 0.1-2% of total zinc. Thus, for example, a formulation with 1% ZnO will have about 0.8% total zinc. The zinc ion may be provided from multiple sources, e.g., about 1% of zinc oxide or about 0.5% zinc oxide together with about 2% zinc citrate. These amounts refer to total zinc in the toothpaste. The zinc in the products of the invention is concentrated in the silica gel portion of the toothpaste, so where the silica gel portion comprises, e.g., 20% of the total toothpaste, and an overall concentration of zinc in the toothpaste is desired to be, e.g., 1%, the silica gel portion will comprise 5% zinc.

The compositions of the invention may comprise various agents which are active to protect and enhance the strength and integrity of the enamel and tooth structure and/or to reduce bacteria and associated tooth decay and/or gum disease, including or in addition to the zinc ion sources. Effective concentration of the active ingredients used herein will depend on the particular agent, the delivery system used, and the exact salt or polymer selected. For example, where the active agent is provided in salt form, the counterion will affect the weight of the salt, so that if the counterion is heavier, more salt by weight will be required to provide the same concentration of active ion in the final product. In some embodiments, the dentifrice may comprise arginine, which where present, may be present at levels from, e.g., about 0.1 to about 20 wt %(expressed as weight of free base), e.g., about 1 to about 10 wt % for a consumer toothpaste or about 7 to about 20 wt % for a professional or prescription treatment product. Fluoride where present may be present at levels of, e.g., about 25 to about 25,000 ppm, for example about 750 to about 2,000 ppm for a consumer toothpaste, or about 2,000 to about 25,000 ppm for a professional or prescription treatment product. Other antibacterial agents may be present in effective amounts, e.g., triclosan may be present in a concentration of about 0.3 wt %.

*Fluoride Ion Source:* The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al. Representative fluoride ion sources include, but are not limited to, stannous fluoride, sodium fluoride, potassium fluoride, sodium rnonofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. In certain embodiments, the oral care composition of the invention may also contain a source of fluoride ions or fluorine-providing ingredient in amounts sufficient to supply about 25 ppm to about 25,0000 ppm of fluoride ions, generally at least about 500 ppm, e.g., about 500 to about 2000 ppm, e.g., about 1000 to about 1600 ppm, e.g., about 1450 ppm, The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride. Fluoride ion sources may be added to the compositions of the invention at a level of about 0.01 wt. % in about 10 wt. % in one embodiment or about 0.03 wt. % to about 5 wt. %. and in another embodiment about 0.1 wt. % to about 1 wt. % by weight of the composition in another embodiment. Weights of fluoride salts to provide the appropriate level of fluoride ion will obviously vary based on the weight of the counterion in the salt.

*Abrasives:* The toothpastes of the invention, e.g. the toothpastes used in Product 1, et seq. may include silica abrasives, e.g., as a component of the silica gel portion. The calcium carbonate abrasive may include precipitated calcium carbonate (e.g. prepared from reaction of calcium oxide with water and carbon dioxide), natural refined calcium carbonate (e.g., from mineral sources), or mixtures thereof. Natural calcium carbonate may have a somewhat harder, more crystalline structure than precipitated calcium carbonate, and in a particular embodiment, the calcium carbonate abrasive is a mixture of the two. The calcium carbonate base portion may also comprise silica abrasives and/or additional abrasives, e.g., a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate; or abrasives such as sodium metaphosphate, potassium metaphosphate. aluminum silicate, calcined alumina, bentonite or other siliceous materials, or combinations thereof.

*Foaming agents:* The oral care compositions of the invention also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Polyox® is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions of the present invention. Where present, the amount of of foaming agent in the oral care composition (i.e., a single dose) is about 0.01 to about 0.9 % by weight, about 0.05 to about 0.5% by weight, and in another embodiment about 0.1 to about 0.2 % by weight.

*Surfactants:* The first and second dentifrice in the products of the invention may comprise one or more surfactants, e,g., selected from anionic, cationic, zwitterionic, and nonionic surfactants, and mixtures thereof, e.g., comprising an anionic surfactant, e.g., a surfactant selected from sodium lauryl sulfate, sodium ether lauryl sulfate, and mixtures thereof, e.g., in an amount of from about 0.3% to about 4.5% by weight. The compositions useful in the invention may contain anionic surfactants, for example:
i. water-soluble salts of higher fatty acid monoglyceride monosulfates, such as the sodium salt of the monosulfated monoglyceride of hydrogenated coconut oil fatty acids such as sodium N-methyl N-cocoyI taurate, sodium cocomonoglyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K, for example sodium laureth-2 sulfate (CH₃(CH₂)₁₀CH₂(OCH₂CH₂)₂OS₃Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C₆₋₃₀ alkyl. In particular embodiments, the anionic surfactant is selected from sodium lauryl sulfate and sodium ether lauryl sulfate. The anionic surfactant may be present in an amount which is effective, e.g., > 0.01% by weight of the formulation, but not at a concentration which would be irritating to the oral tissue, e.g., <10%, and optimal concentrations depend on the particular formulation and the particular surfactant. For example, concentrations used or a mouthwash are typically on the order of one tenth that used for a toothpaste. In one embodiment, the anionic surfactant is present in a toothpaste at from about 0.3% to about 4.5% by weight, e.g., about 1.5%. The compositions of the invention may optionally contain mixtures of surfactants, e.g., comprising anionic surfactants and other surfactants that may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al. In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sareosinate and sodium coconut monoglyceride sulfonates are examples of anionic surfactants of this type. In a particular embodiment, the first and second dentifrices of Product 1, et seq., each comprise sodium lauryl sulfate in an amount of 1-3%, e.g., about 2.25%. The surfactant or mixtures of compatible surfactants can be present in the compositions of the present invention in about 0.1% to about 5.0%, in another embodiment about 0.3% to about 3.0% and in another embodiment about 0.5% to about 2.0% by weight of the total composition.

*Tartar control agents:* In various embodiments of the present invention, the compositions comprise an anticalculus (tartar control) agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. The invention thus may comprise phosphate salts. In particular embodiments, these salts are alkali phosphate salts. i.e., salts of alkali metal hydroxides or alkaline earth hydroxides, for example, sodium, potassium or calcium salts. "Phosphate'" as used herein encompasses orally acceptable mono- and polyphosphates, for example, P₁₋₆ phosphates, for example monomeric phosphates such as monobasic, dibasic or tribasic phosphate; dimeric phosphates such as pyropbosphates; and multimeric phosphates, e.g., sodium hexametaphosphate. In particular examples, the selected phosphate is selected from alkali dibasic phosphate and alkali pyrophosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these. In a particular embodiment, for example the compositions comprise a mixture of tetrasodium pyrophosphate (Na₄P₂O₇), calcium pyrophosphate (Ca₂P₂O₇), and sodium phosphate dibasic (Na₂HPO₄), e.g., in amounts of ca. 3-4% of the sodium phosphate dibasic and ca. 0.2-1 % of each of the pyrophosphates. In another embodiment, the compositions comprise a mixture of tetrasodium pyrophosphate (TSPP) and sodium tripolyphosphate (STPP)(Na₅P₃O₁₀), e.g., in proportions of TSPP at about 1-2% and STPP at about 7% to about 10%. Such phosphates are provided in an amount effective to reduce erosion of the enamel, to aid in cleaning the teeth, and/or to reduce tartar buildup on the teeth, for example in an amount of 2-20%, e.g., ca. 5-15%, by weight of the composition.

*Flavoring Agents:* The oral care compositions of the invention may also include a flavoring agent. Flavoring agents which are used in the practice of the present invention include,but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint. The flavoring agent may be incorporated in the oral composition at a concentration of about 0.1 to about 5% by weight e.g. about 0.5 to about 1.5% by weight.

*Polymers:* The oral care compositions of the invention may also include additional polymers to adjust the viscosity of the formulation or enhance the solubility of other ingredients. Such additional polymers include polyethylene glycols, polysaccharides (e.g., cellulose derivatives, for example carboxymethyl cellulose (CMC), or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts.

Silica thickeners, which form polymeric structures or gels in aqueous media, may be present. Note that these silica thickeners are physically and functionally distinct from the particulate silica abrasives also present in the compositions, as the silica thickeners are very finely divided and provide little or no abrasive action. Other thickening agents are carboxyvinyl polymers, carrageenan, hydroxyethyl cellulose and water soluble salts of cellulose ethers such as sodium carboxymethyl cellulose and sodium carboxymethyl hydroxyelhyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of about 0.5% to about 5.0% by weight of the total composition are used.

The compositions of the invention may include an anionic polymer, for example in an amount of from about 0.05 to about 5%, e.g., in the silica gel portion of the toothpaste. Such agents are known generally for use in dentifrice, although not for this particular application, useful in the present invention are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturatedmonomer, preferably methyl vinyl ether/maleic anhydridehaving a molecular weight (M.W.) of about 30,000 to about 1,000.,000, most preferably about 300,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from about 0.05 to about 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpba-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid. Another useful class of polymeric agents includes polyamino acids containing proportions of anionic surface-active amino acids such as aspartic acid, glutamic acid and phosphoserine, e.g. as disclosed in U.S. Pat. No. 4,866,161 Sikes et al.

*Water:* The oral compositions may comprise significant levels of water. Water employed in the preparation of commercial oral compositions should be deionized and free of organic impurities. The amount of water in the compositions includes, the free water which is added plus that amount which is introduced with other materials.

*Humectants.* Within certain embodiments of the oral compositions, it is also desirable to incorporate a humectant to prevent the composition from hardening upon exposure to air. Certain humectants can also impart desirable sweetness or flavor to dentifrice compositions. Suitable humectants include edible polyhydric alcohols such as glycerine, sorbitol, xylitol, propylene glycol as well as other polyols and mixtures of these humectants. In one embodiment of the invention, the principal humectant is glycerin, which may be present at levels of greater than 25%, e.g. 25-35% about 30%, with 5% or less of other humectants.

*Other optional ingredients:* In addition to the above-described components, the embodiments of this invention can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5,004,597, to Majeti; U.S. Pat. No. 3,959,458 to Agricola et al. and U.S. Pat. No. 3,937,807, to Haefele.

Unless stated otherwise, all percentages of composition components given in this specification are by weight based on a total composition or formulation weight of 100%. In the case of Product 1, et seq., the total composition or formulation weight is the weight of the first and second dentifrices combined.

Unless otherwise specifically identified, the ingredients for use in the compositions and formulations of the present invention are preferably cosmetically acceptable ingredients. By "cosmetically acceptable" is meant suitable for use in a formulation for topical application to human skin. A cosmetically acceptable excipient, for example, is an excipient which is suitable for external application in the amounts and concentrations contemplated in the formulations of this invention, and includes for example excipients which are "Generally Recognized as Safe" (GRAS) by the United States Food and Drug Administration.

The compositions and formulations as provided herein are described and claimed with reference to their ingredients, as is usual in the art. As would be evident to one skilled in the art, the ingredients may in some instances react with one another, so that the true composition of the final formulation may not correspond exactly to the ingredients listed. Thus, it should be understood that the invention extends to the product of the combination of the listed ingredients.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

### EXAMPLES

### Example 1

The following test formulations are prepared:
(i) a white active salt dentifrice base, comprising calcium carbonate abrasive but no zinc;
(ii) the dentifrice base of (i) modified to include 1% zinc oxide,
(iii) the dentifrice base of (i) modified to include 0.5% zinc oxide and 2% zinc citrate, and
(iv) a dual component dentifrice which is 80% a dentifrice base (corresponding to the first dentifrice on Product 1, *supra*) having substantially the same as (i) and 20% a gel dentifrice stripe (corresponding to the second dentifrice of Product 1, *supra*) without calcium carbonate but with 5% zinc oxide, i,e, having the same total amount of zinc oxide as (ii), but in the silica gel rather than in the calcium carbonate base.

The specific ingredients of the test formulations are as set forth in Table 1:

**Table 1. Calcium Carbonate Based Dentifrice Containing Zinc Salts (AS = Active Calcium Carbonate Salt, all ingredients w/w%)**

| **Description** | **AS Base** | **AS w/1 % ZnO** | **AS w/0.5% ZnO+ 2% Zn Cit** | **AS 80: 20 stripe** |
|---|---|---|---|---|
| Ingredients (%) | | | | *AS base: 80% ingredients by weight of AS base component)* |
| Sorbitol 70% | 21 | 21 | 21 | 21 |
| Xanthan | 0.2 | 0.2 | 0.2 | 0.2 |
| CMC TMS | 0.72 | 0.72 | 0.72 | 0.72 |
| Sodium Chloride | 0.5 | 0.5 | 0.5 | 0.63 |
| Sodium Saccharin | 0.27 | 0.27 | 0.27 | 0.27 |
| Sodium Silicate | 1.2 | 1.2 | 1.2 | 1.2 |
| Sodium Bicarbonate | 0.5 | 0.5 | 0.5 | 0.63 |
| Thickening Silica | 2.5 | 2.5 | 2.5 | 2.5 |
| Precipitated Calcium Carbonate | 18 | 18 | 18 | 18 |
| Refined Natural Calcium Carbonate | 22.5 | 22.5 | 22.5 | 22.5 |
| Tetrasodium pyrophosphate | -- | 0.5 | 0.5 | -- |
| Sodium Monofluorophosphate | 0.76 | 0.76 | 0.76 | 0.76 |
| Sodium Lauryl Sulfate Granules | 2.2 | 2.25 | 2.25 | 2.25 |
| Flavor | 1 | 1 | 1 | 1 |
| Purified Water | q.s | q.s | q.s | q.s |
| Zinc Oxide | -- | 1 | 0.5 | -- |
| Zinc Citrate | -- | -- | 2 | -- |

| | | | | *Blue Get Stripe: 20% (ingredients by weight of gel component)* |
|---|---|---|---|---|
| Sorbitol 70 % | -- | -- | -- | 40 |
| Xanthan | -- | -- | -- | 0.2 |
| CMC TMS | -- | -- | -- | 0.72 |
| Sodium Saccharin | -- | -- | -- | 0.27 |
| Thickening Silica | -- | -- | -- | 1.5 |
| Abrasive Silica | -- | -- | -- | 20 |
| Sodium Monofluorophosphate | -- | -- | -- | 0.76 |
| Sodium Lauryl Sulfate Granules | -- | -- | -- | 2,25 |
| Flavor | -- | -- | -- | 1 |
| Purified Water | -- | -- | -- | q.s |
| Zinc Oxide | -- | -- | -- | 5 |
| Pigment Blue | -- | -- | -- | 0.05 |

### Example 2

The touthpastes of Example 1 are evaluatede as follows:
1. Saliva is collected from 4 healthy volunteers and pooled together as inoculum.
2. Sterile HAP disks are incubated under anaerobic conditions at 37 °C for 24 hours with 1 ml of sterile artificial saliva (with 0.01% sucrose) and 1 ml of pooled saliva in a 24 well microplate.
3. Freshly prepared treatment solution 1 part dentifrice: 2 parts distilled water is added to the well and allowed to contact with the HAP disk for 10 minutes.
4. The liquid phase is removed and replaced by 2 ml of sterile artificial saliva.
5. The disks are treated in triplicates for each control and test dentifrice for 8 days
6. At intervals of 2, 4 and 8 days the discs are collected aseptically and transferred into prereduced thioglycollate medium.
7. 0.01ml of the dilution 10-4, 10-5 and 10-6 are plated in triplicate for each disk on the following media:
   a) Neomycin -Vancomycin (NV) Agar for Total Gram negative Anaerobes.
8. Plates are surface spread using a sterile spreader and incubated anaerobically @37°C for 72 hours before counting the colonies.
9. The pH is monitored for the entire period of the study using the liquid phase.

Plate counts are recorded in log reduction as in Table 2:

**Table 2: Average Log CFU/ml**

| | Day 2 | | Day 4 | | Day 8 | |
|---|---|---|---|---|---|---|
| Neomycin Vancomycin Agar | Avg Log CFU | SEM | Avg Log CFU | SEM | Avg Log CFU | SEM |
| Active Salt Base | 7.21 | 0,0380 | 7.54 | 0.0325 | 7.75 | 0.0268 |
| CAS w/1% ZnO | 7.26 | 0.0179 | 6.67 | 0.0205 | 5.83 | 0.0276 |
| CAS w/0.5% XnO +2% Zn Cit | 7.31 | 0.0382 | 6.46 | 0.0334 | 5.67 | 0.0248 |
| CAS 80:20 stripe (Chalk: Silica) | 7.23 | 0.0379 | 6.43 | 0.0381 | 5.41 | 0.0382 |

It is observed that the localization of ZnO in the silica stripe further enhances the bacterial reduction attributes of a calcium carbonate dentifrice.

ANOVA is done for the data at 95% significance level as set forth in Table 3.

**Table 3: Descriptive Statistics**

| Treatment | Day 2 | | | Day 4 | | | Day 8 | | |
|---|---|---|---|---|---|---|---|---|---|
| | Mean | SE Mean | St.Dev | Mean | SE Mean | St.Dev | Mean | SE Mean | St.Dev |
| Active Sail Base | 7.205 | 0.040 | 0.119 | 7.536 | 0.032 | 0.097 | 7.749 | 0.035 | 0.104 |
| CAS 80:20 stripe (Chalk: Silica) | 7.229 | 0.040 | 0.121 | 6.429 | 0.042 | 0.127 | 5.408 | 0.037 | 0.112 |
| CAS w/1% ZnO | 7.260 | 0.026 | 0.078 | 6.668 | 0.021 | 0.062 | 5.830 | 0.030 | 0.089 |
| CAS w/0.5% ZnO + 2% Zn Cit | 7.315 | 0.041 | 0.123 | 6.458 | 0.036 | 0.108 | 5.693 | 0.032 | 0,095 |

**Table 4: Significance table**

| Treatment | Day 2 | Day 4 | Day 8 |
|---|---|---|---|
| Active Salt Base | No significant differences between treatments | A | A |
| CAS80:20 stripe (Chalk: Silica) | | D | E |
| CAS w/1% ZnO | | C | C |
| CAS w/0.5% ZnO +2% Zn Cit | | D | CD |

Statistical analysis further confirms the difference in efficacy observed.

## Claims

1. A toothpaste product comprising
a first dentifrice comprising a calcium carbonate abrasive,
a second dentifrice comprising a zinc ion source in a gel base, and
a container which holds the first dentifrice physically separate from the second dentifrice until the dentifrices are dispensed,
wherein the amount of first dentifrice relative to the second dentifrice is 4:1.

2. The product of claim 1, wherein the product is capable of dispensing the second dentifrice as a stripe entrained in the first dentifrice.

3. The toothpaste product of claim 1 or claim 2, wherein the zinc ion source is selected from zinc oxide, zinc citrate, and mixtures thereof.

4. The toothpaste product of any of the foregoing claims wherein the second dentifrice contains zinc in an amount of 0.1 to 10% by weight of the second dentifrice.

5. The toothpaste product of any of the foregoing claims wherein the second dentifrice contains a zinc ion source selected from (i) zinc oxide in an amount of 0.1 to 6%; (ii) zinc citrate in an amount of 0.1 to 10%; and (iii) zinc oxide in an amount of 0.1 to 6% and zinc citrate in an amount of 0.1 to 10%.

6. The toothpaste product of any of the foregoing claims wherein the the second dentifrice contains about 5% zinc oxide.

7. The toothpaste product of any of the foregoing claims wherein the second dentifrice comprises a viscosity-modifying amount of one or more thickening agents selected from carboxymethyl cellulose, xanthan, thickening silica, and mixtures thereof.

8. The toothpaste product of any of the foregoing claims wherein the calcium carbonate abrasive in the first dentifrice is a mixture of precipitated calcium carbonate and natural calcium carbonate in an amount of 35-45% by weight of the first dentifrice.

9. The toothpaste product of any of the foregoing claims wherein the second dentifrice comprises silica abrasive in an amount of 10-40% by weight of the second dentifrice.

10. The toothpaste product of any of the foregoing claims wherein the first dentifrice is an opaque white paste and the second abrasive is a colored translucent gel.

11. A striped toothpaste comprising a first dentifrice comprising a calcium carbonate abrasive, second dentifrice comprising a zinc ion source in a gel base, wherein the amount of first dentifrice relative to the second dentifrice is 4:1, wherein the second dentifrice is entrained as a stripe in the first dentifrice.

12. A method of making a dentifrice comprising a first dentifrice comprising a calcium carbonate abrasive and a second dentifrice comprising a zinc ion source in a gel base, wherein the amount of first dentifrice relative to the second dentifrice is 4:1, comprising entraining the second dentifrice as a stripe in the first dentifrice.

13. The product according to any one of claims 1 to 11, for use in providing an antibacterial benefit to the oral cavity of a subject in need thereof.

## Patentansprüche

1. Zahnpastaprodukt, umfassend
ein erstes Zahnreinigungsmittel, umfassend einen Calciumcarbonat-Abrasivstoff,
ein zweites Zahnreinigungsmittel, umfassend eine Zink-Ionenquelle in einer Gel-Base, und
einen Behälter, der das erste Zahnreinigungsmittel physikalisch getrennt von dem zweiten Zahnreinigungsmittel aufnimmt, bis die Zahnreinigungsmittel abgegeben werden,
wobei die Menge des ersten Zahnreinigungsmittels relativ zu der des zweiten Zahnreinigungsmittels 4:1 ist.

2. Produkt nach Anspruch 1, wobei das Produkt in der Lage ist, das zweite Zahnreinigungsmittel als Streifen, aufgenommen in das erste Zahnreinigungsmittel, abzugeben.

3. Zahnpastaprodukt nach Anspruch 1 oder Anspruch 2, wobei die Zink-Ionenquelle ausgewählt ist aus Zinkoxid, Zinkcitrat und Mischungen davon.

4. Zahnpastaprodukt nach irgendeinem der vorhergehenden Ansprüche, wobei das zweite Zahnreinigungsmittel Zink in einer Menge von 0,1 bis 10 Gew.-% des zweiten Zahnreinigungsmittels enthält.

5. Zahnpastaprodukt nach irgendeinem der vorhergehenden Ansprüche, wobei das zweite Zahnreinigungsmittel eine Zink-Ionenquelle enthält, ausgewählt aus (i) Zinkoxide in einer Menge von 0,1 bis 6%; (ii) Zinkcitrat in einer Menge von 0,1 bis 10%; und (iii) Zinkoxid in einer Menge von 0,1 bis 6% und Zinkcitrat in einer Menge von 0,1 bis 10%.

6. Zahnpastaprodukt nach irgendeinem der vorhergehenden Ansprüche, wobei das zweite Zahnreinigungsmittel etwa 5% Zinkoxid enthält.

7. Zahnpastaprodukt nach irgendeinem der vorhergehenden Ansprüche, wobei das zweite Zahnreinigungsmittel eine die Viskosität modifizierende Menge von einem oder mehreren Verdickungsmitteln umfasst, ausgewählt aus Carboxymethylcellulose, Xanthan, verdickendes Silica und Mischungen davon.

8. Zahnpastaprodukt nach irgendeinem der vorhergehenden Ansprüche, wobei der Calciumcarbonat-Abrasivstoff in dem ersten Zahnreinigungsmittel eine Mischung aus ausgefälltem Calciumcarbonat und natürlichem Calciumcarbonat in einer Menge von 35-45 Gew.-% des ersten Zahnreinigungsmittels ist.

9. Zahnpastaprodukt nach irgendeinem der vorhergehenden Ansprüche, wobei das zweite Zahnreinigungsmittel Silica-Abrasivstoff in einer Menge von 10-40 Gew.-% des zweiten Zahnreinigungsmittels umfasst.

10. Zahnpastaprodukt nach irgendeinem der vorhergehenden Ansprüche, wobei das erste Zahnreinigungsmittel eine opake weiße Paste ist und das zweite Abrasivmittel ein gefärbtes transluzentes Gel ist.

11. Gestreifte Zahnpasta, umfassend ein erstes Zahnreinigungsmittel, umfassend einen Calciumcarbonat-Abrasivstoff, zweites Zahnreinigungsmittel, umfassend eine Zink-Ionenquelle in einer Gel-Base, worin die Menge des ersten Zahnreinigungsmittels relativ zu der des zweiten Zahnreinigungsmittels 4:1 ist, wobei das zweite Zahnreinigungsmittel als ein Streifen in dem ersten Zahnreinigungsmittel aufgenommen ist.

12. Verfahren zur Herstellung eines Zahnreinigungsmittels, umfassend ein erstes Zahnreinigungsmittel, umfassend einen Calciumcarbonat-Abrasivstoff und ein zweites Zahnreinigungsmittel, umfassend eine Zink-Ionenquelle in einer Gel-Base, worin die Menge des ersten Zahnreinigungsmittels relativ zu der des zweien Zahnreinigungsmittels 4:1 ist, umfassend das Aufnehmen des zweiten Zahnreinigungsmittels als ein Streifen in dem ersten Zahnreinigungsmittel.

13. Produkt nach irgendeinem der Ansprüche 1 bis 11 zur Verwendung bei der Bereitstellung eines antibakteriellen Vorteils an die Mundhöhle eines bedürftigen Subjektes.

## Revendications

1. Produit dentifrice comprenant
un premier dentifrice comprenant un abrasif à base de carbonate de calcium,
un second dentifrice comprenant une source d'ions zinc dans une base de gel, et
un récipient qui retient le premier dentifrice physiquement séparé du second dentifrice jusqu'à ce que les dentifrices soient distribués,
dans lequel la quantité du premier dentifrice par rapport au second dentifrice est de 4:1.

2. Produit selon la revendication 1, dans lequel le produit est apte à distribuer le second dentifrice sous la forme d'une bande entraînée dans le premier dentifrice.

3. Produit dentifrice selon la revendication 1 ou la revendication 2, dans lequel la source d'ions zinc est choisie parmi l'oxyde de zinc, le citrate de zinc et leurs mélanges.

4. Produit dentifrice selon l'une quelconque des revendications précédentes, dans lequel le second dentifrice contient du zinc en une quantité de 0,1 à 10 % en poids du second dentifrice.

5. Produit dentifrice selon l'une quelconque des revendications précédentes, dans lequel le second dentifrice contient une source d'ions zinc choisie parmi (i) de l'oxyde de zinc en une quantité de 0,1 à 6 % ; (ii) du citrate de zinc en une quantité de 0,1 à 10 % ; et (iii) de l'oxyde de zinc en une quantité de 0,1 à 6 % et du citrate de zinc en une quantité de 0,1 à 10 %.

6. Produit dentifrice selon l'une quelconque des revendications précédentes, dans lequel le second dentifrice contient environ 5 % d'oxyde de zinc.

7. Produit dentifrice selon l'une quelconque des revendications précédentes, dans lequel le second dentifrice comprend une quantité modifiant la viscosité d'un ou plusieurs agents épaississants choisis parmi la carboxyméthylcellulose, le xanthane, une silice épaississante et leurs mélanges.

8. Produit dentifrice selon l'une quelconque des revendications précédentes, dans lequel l'abrasif à base de carbonate de calcium dans le premier dentifrice est un mélange de carbonate de calcium précipité et de carbonate de calcium naturel en une quantité de 35 à 45 % en poids du premier dentifrice.

9. Produit dentifrice selon l'une quelconque des revendications précédentes, dans lequel le second dentifrice comprend un abrasif à base de silice en une quantité de 10 à 40 % en poids du second dentifrice.

10. Produit dentifrice selon l'une quelconque des revendications précédentes, dans lequel le premier dentifrice est une pâte blanche opaque et le second abrasif est un gel translucide coloré.

11. Dentifrice à rayures comprenant un premier dentifrice comprenant un abrasif à base de carbonate de calcium, un second dentifrice comprenant une source d'ions zinc dans une base de gel, dans lequel la quantité du premier dentifrice par rapport au second dentifrice est de 4:1, dans lequel le second dentifrice est entraîné sous la forme d'une bande dans le premier dentifrice.

12. Procédé de préparation d'un dentifrice comprenant un premier dentifrice comprenant un abrasif à base de carbonate de calcium et un second dentifrice comprenant une source d'ions zinc dans une base de gel, dans lequel la quantité du premier dentifrice par rapport au second dentifrice est de 4:1, comprenant l'entraînement du second dentifrice sous la forme d'une bande dans le premier dentifrice.

13. Produit selon l'une quelconque des revendications 1 à 11, pour une utilisation dans la fourniture d'un bénéfice antibactérien à la cavité buccale d'un sujet en ayant besoin.
